Europäisches Patentamt

European Patent Office

Office européen des brevets

(19)

(11) EP 0 785 217 A1

(12) **EUROPEAN PATENT APPLICATION**

(43) Date of publication:
**23.07.1997 Bulletin 1997/30**

(21) Application number: **97400098.6**

(22) Date of filing: **17.01.1997**

(51) Int Cl.$^6$: **C07K 16/40**, G01N 33/577,
G01N 33/573
// C12N9/90

(84) Designated Contracting States:
**DE FR GB**

(30) Priority: **19.01.1996 JP 24897/96**
**04.12.1996 JP 337723/96**

(71) Applicant: **ORIENTAL YEAST CO., LTD.**
**Tokyo (JP)**

(72) Inventors:
• **Uchida, Kohji**
**Hikone-shi, Shiga-ken (JP)**
• **Matuo, Yushi**
**Suita-shi, Osaka-fu (JP)**

• **Tanigushi, Yoshiyuki**
**Izumiotsu-shi, Osaka-fu (JP)**
• **Mori, Kyoko**
**Hikone-shi, Shiga-ken (JP)**
• **Kasuya, Kenji**
**Ibaraki-shi, Osaka-fu (JP)**
• **Fujita, Tuyosi**
**Ikeda-shi, Osaka-fu (JP)**

(74) Representative: **Clisci, Serge et al**
**S.A. FEDIT-LORIOT & AUTRES**
**CONSEILS EN PROPRIETE INDUSTRIELLE**
**38, Avenue Hoche**
**75008 Paris (FR)**

(54) **Method of immunoassay for human BPGM**

(57) The invention relates to the novel production of recombinant human BPGM (bisphosphoglycerate mutase) through genetic engineering operation with *E. coli,* the novel specific antibody to BPGM as prepared by the use of the recombinant human BPGM as an antigen, and the quantitative determination of BPGM in a sample by the use of the antibody. Since the antibody of the invention has high specificity, the accurate determination of BPGM is possible. The antibody can be used in diagnosis of various diseases.

EP 0 785 217 A1

**Description**

DETAILED DESCRIPTION OF THE INVENTION:

Technical Field of the Invention:

The present invention relates to an antibody to bisphosphoglycerate mutase (BPGM), and to a system of measuring BPGM using the antibody.

Prior Art:

The group of isozymes of mammalian phosphoglycerate mutase (PGAM) includes B-type PGAM (non-muscular isozyme) composed of BB homodimers, M-type PGAM (muscular isozyme) composed of MM homodimers, MB-type PGAM (myocardial isozyme) composed of MB heterodimers, and in addition to these, bisphosphoglycerate mutase (BPGM; EC5.4.2.4, formerly EC2.7.5.4) composed of EE homodimers that specifically exists in red blood cells, and these isozymes have different subunit constitutions.

BPGM catalyzes the synthesis and decomposition of 2,3-bisphosphoglycerate (2,3-BPG). The subunit E constituting BPGM has a molecular weight of about 30,000 and is about 50 % homologous to subunits B and M.

2,3-BPG is a co-factor in PGAM reaction, while playing an important role in red blood cells as a factor that regulates the oxygen affinity of hemoglobin. Precisely, 2,3-BPG exists in red blood cells of human and other many mammals at a high concentration, while directly binding to hemoglobin to lower its oxygen affinity thereby promoting the supply of oxygen into tissue. In addition, it lowers the pH in red blood cells, thereby lowering the oxygen affinity of hemoglobin.

Some diseases that express abnormal levels of 2,3-BPG in red blood cells have heretofore been reported. For example, diseases that increase 2,3-BPG include hemolytic anemia, iron deficiency anemia, cyanotic congenital cardiopathy, congestive failure, chronic pneumonopathy, hepatocirrhosis, renal insufficiency, uremia, and hyperthyroidism; while those that decrease 2,3-BPG include diabetic ketoacidosis, hexokinase deficiency, and hereditary spherocytosis.

Therefore, it is very useful to establish the method of quantitative determination of BPGM that participates in the synthesis and decomposition of 2,3-BPG, and BPGM is expected, for example, as a good marker for hemolytic disorders.

On the other hand, immunoassay which is one important assay method in clinical examinations depends on the quality of antigen and that of antibody used. In this, however, where human-derived antigens are used, it is difficult to stably supply high-purity human antigens for the following reasons. i) Raw materials for human antigens are difficult to obtain. ii) The antigen content of raw materials is generally low. iii) If raw materials of (patients') sera are used, there is a great risk of their being contaminated with viruses. With the recent development in the genetic recombination technology, it has become possible to prepare large amounts of high-purity, human-derived proteins and enzymes. Therefore, it may be possible to overcome the above-mentioned drawbacks by using recombinant proteins in place of human-derived antigens that have heretofore been used in immunoassay. In addition, it has become possible to develop novel methods for clinical examination by intentionally employing proteins and isozymes that have heretofore been difficult to prepare.

In fact, however, the practical use of such recombinant proteins in this technical field in place of human-derived proteins and enzymes is often delayed for the reasons that the recombinant proteins prepared do not have physiological activity and that the amount of the recombinant proteins to be expressed is small. In particular, for proteins with specific physiological activity, such as enzymes, it is necessary to develop the technique capable of efficiently preparing high-purity recombinant proteins as active proteins having higher-order structures but not as unfolding proteins that may be expressed in the form of inclusion bodies.

Problems to be Solved by the Invention:

In consideration of the background art mentioned above, the present invention has been made for the purpose of expressing recombinant human BPGM with enzymatic activity through genetic engineering operation and also to prepare a specific antibody to the enzyme, thereby newly developing novel immunoassay for BPGM in red blood cells or plasma with high sensitivity and high specificity thereto.

Brief Description of the Drawings:

Fig. 1 shows the nucleotide sequence and the amino acid sequence of the primer used herein.
Fig. 2 shows the results of the analysis through SDS-PAGE in the step of purification of the recombinant human BPGM obtained from an extract of cells of *E. coli* (pTRP-PBGM2).

Fig. 3 graphically shows the principle of the quantitative determination of human BPGM by EIA.

Fig. 4 shows the calibration curve of BPGM.

Fig. 5 shows the specificity of human PBGM-specific IgG.

Fig. 6 shows the dilution linearity in hemolyzed samples.

Fig. 7 shows a map for constructing an expression vector, pTRP.

Fig. 8 shows a gene cassette for tryptophan promoter.

Fig. 9-(1) to Fig. 9-(5) show influence of interference substances on assay of BPGM.

Means for Solving the Problems:

In order to attain the above-mentioned object, we, the present inventors have assiduously studied from various aspects, and have succeeded in, for the first time, the effective production of recombinant human BPGM through genetic engineering operation. In addition, after having immunized rabbits with an antigen of the thus-obtained pure PBGM, we have also succeeded in collecting IgG that is specific to BPGM, from the anti-serum of the immunized rabbits. As a result of further studies on these, we have completed the present invention.

Now, the present invention is described in detail hereinunder.

The recombinant human BPGM which is used as the antigen is expressed by inserting a human BPGM gene into an expression vector having a trp promoter to construct a human BPGM-expressing plasmid, then transforming microorganisms, such as *E. coli*, with this plasmid, and incubating the resulting transformant.

Next, the thus-obtained human BPGM is purified, and animals, such as rabbits, are immunized with this as an antigen. The anti-serum obtained from the immunized animals is purified through chromatography or the like to obtain the intended, purified polyclonal antibody with high specificity.

The thus-obtained antibody is novel and has been confirmed to have extremely high specific reactivity with the antigen, human BPGM. On the basis of these findings followed by further studies thereon, we, the present inventors have completed the present invention.

In the present invention, human BPGM is immunoassayed using the thus-prepared antibody. To the immunoassay, appropriately applicable are ordinary methods of, for example, immunostaining assay, radioimmunoassay (RIA), enzyme immunoassay (EIA) and fluorescent immunoassay.

Of these, solid-phase immunoassay is advantageously used. For example, in two-antibody immunoassay, a solid-phase BPGM IgG is reacted with a sample, which is then reacted with a biotinated BPGM IgG, and thereafter with an avidin-biotinated enzyme. From the data resulting from the reaction with the enzyme, the amount of BPGM in the sample is obtained.

The preparation of the solid-phase antibody can be conducted according to any ordinary method of binding enzymes and other proteins to solid phases. For example, beads, microplates or the like are used as solid phases, to which the antibody is bound according to known fixation techniques for, for example, physical adsorption or chemical bond.

Enzyme-labeled antibodies for EIA can be prepared according to known methods using, for example, avidin-biotin, glutaraldehyde or maleimide. As the labeling enzymes, usable are any enzymes which are generally used in EIA, such as horseradish peroxidase, bovine intestinal alkaline phosphatase, urease, glucose oxidase, and β-galactosidase. The substrates for the enzymes may be any known ones. For example, when alkaline phosphatase is used, p-nitrophenylphosphoric acid is preferably used as the substrate.

Now, examples of the present invention are described below.

Examples:

(1) Construction of BPGM-Expressing Plasmid:

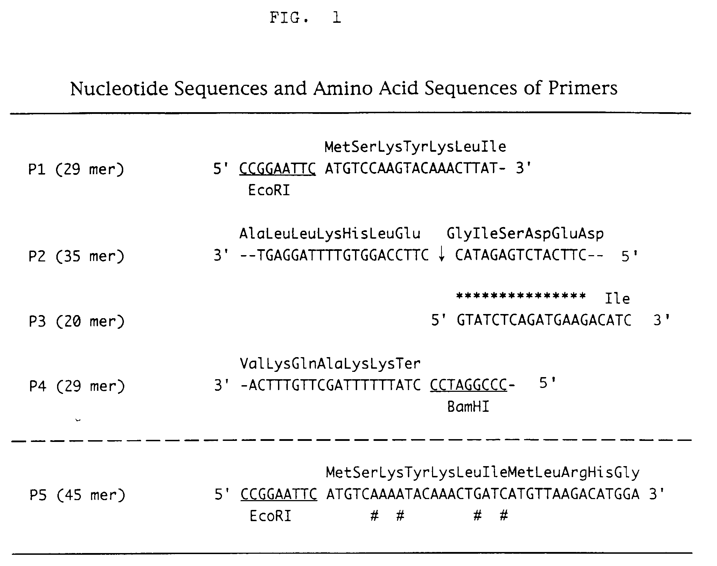

Genetic cloning of human BPGM and the nucleotide sequence thereof were already reported. A DNA coding for human BPGM was prepared by linking at a time two exons of a human BPGM gene through mPCR. Precisely, PCR was conducted, using a human placenta-derived chromosomal DNA as the template and using primers, P1 to P4 shown in Fig. 1. The resulting PCR product was cut with EcoRI and BamHI, and the resulting fragment was inserted into pTRP to construct a BPGM-expressing plasmid, pTRP-BPGM1.

In Fig. 1, the underlined nucleotide sequence indicates the linker for the restriction endonuclease cleavage site. The complementary sequence in primer P3 was marked with *. The base-substituting site in primer P5 was marked with #. The splicing site was marked with an arrow. Ter in the amino acid sequence of P4 means a termination codon.

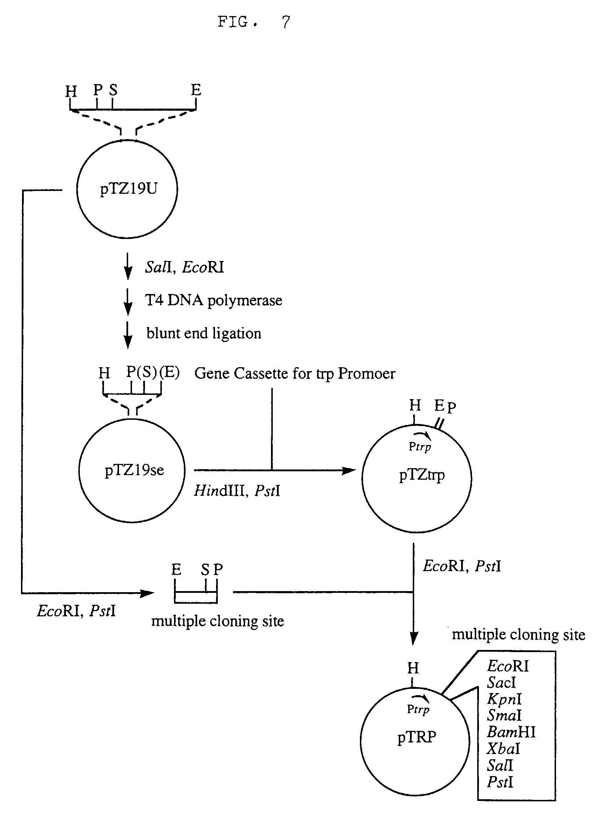

The expression vector pTRP was constructed in the manner mentioned below (see Fig. 7). In Fig. 7, E means EcoRI, H means HindIII, S means SalI, and P means PstI.

i) pTZ19U was cut with SalI and EcoRI, then repaired with a T4 DNA polymerase to make the DNA have a blunt terminal. These were ligated to be re-cycled. Therefore, the plasmid obtained herein, pTZ19se did not have the cloning site between SalI and EcoRI, which the plasmid pTZ19U had.

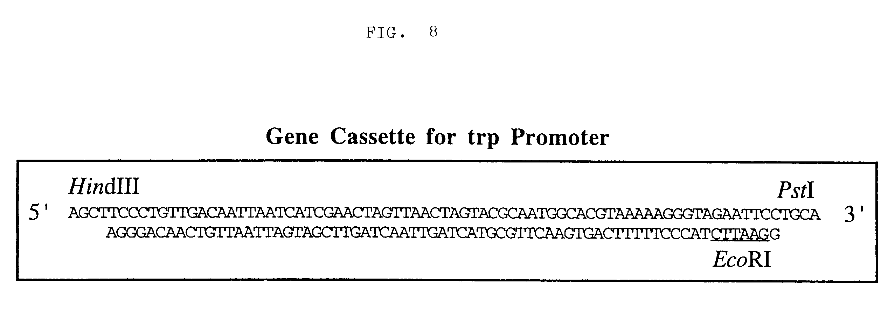

ii) For the gene cassette for *E. coli* trp promoter (see Fig. 8), the sense and the anti-sense were mixed, and inserted into pTZse between the HindIII site and the EcoRI site to prepare pTZtrp.

iii) From pTZ19U, purified was a DNA fragment having a cloning site between EcoRI and PstI, using a GENE CLEAN II kit (BIO 101 Inc.), and this was inserted into the pTZtrp between EcoRI and PstI to prepare an expression vector, pTRP (2928 bp). This pTRP had, as the cloning sites for exogenous genes, EcoRI, SacI, KpnI, SmaI, BamHI, XbaI, SalI and PstI (see Fig. 7).

PCR was conducted according to the method already reported. The faithfulness in all these plasmids constructed and in the entire gene sequence of the BPGM gene was confirmed, using an automatic DNA sequencer. The secondary structure of mRNA was measured and calculated according to a GENETYX program.

(2) Transformation of E. coli and Incubation of Transformant:

Competent cells of *E. coli* JM109 were transformed with the control plasmid and the recombinant plasmid having the BPGM gene. The transformants were incubated in an LB medium containing 50 µg/ml of ampicillin, at 37°C for 24 hours. To prepare a crude extract from the cells, employed was the Marston's bacteriolysis method, except that Lubrol PX was used as the surfactant in place of deoxychloric acid.

To purify the recombinant human BPGM, employed was high-density incubation using a 16L New Brunswick Scientific Fermenter, SF-116 (Edison, NJ, USA). The incubation parameters, temperature (37°C), pH (7.4), revolution (800 rpm), and aeration (15 ml/min) were controlled, using ML-4100 (multiple-loop microprocessor controller). To adjust the pH, added was 10 % ammonia solution. After having been pre-incubated overnight, the culture (240 ml) was transplanted in an LB medium (8 liters) containing 25 µg/ml of ampicillin, and incubated therein. During the incubation for 10 hours, an additional medium totaling 2 liters was continuously added to the culture.

The additional medium comprised of 10 % glucose, 15 % yeast extract, 0.1 % $MgSO_4 \cdot 7H_2O$, 0.75 % L-glutamic acid, 0.75 % L-threonine, 0.04 % L-tyrosine, 0.4 % histidine, and 0.4 % methionine (pH 7.4). The initial rate of the addition of the medium was 100 ml/hr, and the rate was increased to 200 ml/hr, 300 ml/hr and 400 ml/hr in that order at intervals of 2 hours. After 12 hours, the culture was centrifuged (11,000 g x 20 min) to collect the cells. The growth of the cells was checked by measuring the absorbance at 600 nm of the culture.

(3) Measurement of PBGM Activity:

The PBGM activity was measured according to the Calvin et al's method. Precisely, used herein was a mixed liquid of reactants (1 ml in total), which was comprised of 50 mM Tris-HCl (pH 8.0), 7 mM $KH_2PO_4$, 7 mM 1,6-bisphosphofructose, 1 mM NAD, 2 mM 3-phosphoglycerate, 0.38 units/ml aldolase, 10 units/ml triosephosphate isomerase, and 0.8 units/ml glycerate-3-phosphate dehydrogenase. To start the reaction, 50 µl of the enzyme sample was added to this, whereupon the increase in the absorbance at 340 nm ($A_{340}$ nm) for NADH was measured. One unit of the enzyme was defined to be the amount of the enzyme that increased NADH by 1 µmol/min at 37°C in the reaction mixture. To determine the amount of NADH, used was an absorbance coefficient of 6.2 x $10^3$ liter/mole/cm.

(4) Determination and Analysis of Protein:

The amount of the protein produced was determined according to the bicinchoninic acid method using bovine serum albumin as the standard. To analyze the protein, employed was the Laemmli's method, using a commercially-available gel with 10 to 20 % gradient(Dai-ichi Chemical Co.; 84 mm height x 90 mm width x 1 mm thickness), according to SDS-polyacrylamide gel electrophoresis (PAGE). The protein was stained with Coomassie Brilliant Blue R-250 (CBBR). The amino acid sequence of the protein was determined, using a 477A Protein/Peptide Sequencer (ABI Co.) equipped with HPLC Model 120A.

(5) Preparation of Crude Extract from Recombinant Cells:

The cells of JM109 transformed with pTRP-BPGM2 were suspended in 3-fold volume of 50 mM Tris-HCl buffer (pH 7.5). The cells thus suspended were attrited, and 1/50-fold volume of 10 % Lubrol PX was added thereto. Then, the resulting suspension was centrifuged (22,000 g x 30 min, 4°C) to obtain a crude cell extract as the supernatant. The attrition of the cells was conducted, using a DYNO-laboratory mill type KDL (Willy A. Bachofen AG, Basle, Switzerland) (600 ml of grinding elements; 3.3 ml/min continuous operation; grinding beads, MK-IGX with 0.1 to 0.2 mmφ).

(6) Purification of Recombinant BPGM:

The crude extract was heat-treated at 55°C for 30 minutes, and the supernatant resulting from its centrifugation was dialyzed against 10 mM Tris-HCl buffer (pH 7.5) containing 0.1 M NaCl. Next, the resulting dialysate was subjected to affinity chromatography using a Blue-Sepharose column (2.5 x 17 cm) that had been equilibrated with 10 mM Tris-HCl buffer (pH 7.5) containing 0.1 M NaCl. From this, the elution was conducted with 10 mM Tris-HCl buffer (pH 7.5) containing 0.1 M NaC1 and 2 mM 2-phosphoglycerate.

(7) Immunoassay:

According to the Harlow et al's method (Harlow E., Lane D. Antibodies; A Laboratory Manual, Cold Spring Harbor: Cold Spring Harbor Laboratory, 1988), a rabbit anti-BPGM anti-serum was prepared, and IgG was prepared from the serum. EIA herein was conducted according to the Kihira et al's method (Kihira Y., Aiba S., Artificial Immunoglobulin G-binding Protein Mimetic to Staphylococcal Protein A. Its Production and Application to Affinity Purification of Immunoglobulin G., J. Chromatogr., 1992: 597: 277-283). To measure A492 nm, used was an A4 Microplate Reader (Tosoh Corp., Tokyo, Japan). The Western blotting analysis was conducted according to the Towbin et al's method, using a polyvinylidene difluoride membrane. As the secondary antibody, used was a goat anti-rabbit IgG horseradish peroxidase conjugate. The IgG content was obtained, relative to the 1 mg/ml solution with $A_{280}$ = 1.5.

(8) Preparation of B-type PGAM-bound Column:

A solution of pure recombinant B-type PGAM (480 mg) as dissolved in 80 ml of 0.1 M sodium phosphate buffer (pH 7.0) containing 0.15 M NaCl was added to 100 ml of Formyl-Cellulofine (about 77 g of wet gel). This was shaken for 3 hours at room temperature, and 539 mg of trimethylamine borane was added thereto and further shaken overnight at 4°C. Next, 50 ml of 0.1 M hydroxylammonium chloride was added thereto, shaken for 2 hours at room temperature, and filtered. The resulting gel was washed with 20-fold volume of distilled water.

This was further washed with 20-fold volume of PBS and 0.3 M KCl-HCl (pH 2.3) for a total of three times. The ligand-immobilized amount was from 85 to 90 %. The thus-prepared B-type PGAM-Cellulofine gel was filled into a column (5 x 5 cm) to prepare a B-type PGAM-bound column.

(9) Expression of Human BPGM Isozyme in *E. coli*:

The BPGM-expressing plasmid, pTRP-BPGM1 has a DNA coding for human BPGM in the downstream after the trp promoter. The cells of Escherichia coli (pTRP-BPGM1) which is the transformant obtained by transforming E. coli JM109 with this expression plasmid were incubated, and the BPGM activity of the cell extract obtained from these was measured. The BPGM activity of the cell extract from the cells of *E. coli* transformed with the BPGM-expressing plasmid was detected herein, while no such BPGM activity was detected in the control, *E. coli* transformed with pTRP. It is well known that the human enzyme expression in *E. coli* depends on the secondary structure of mRNA. The secondary structure formation at the 5'-region in mRNA of BPGM as produced with the expression plasmid was estimated, and the 5'-coding region of the human PBGM gene was re-constructed while preventing any amino acid substitution therein that might be caused by the secondary structure formation then estimated.

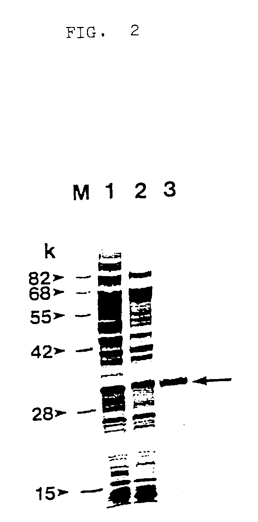

Using P4 and P5 shown in Fig. 1, prepared was pTRP-BPGM2. The cells of Escherichia coli (pTRP-BPGM2) which is the transformant obtained by transforming E. coli JM109 with this plasmid were incubated, and the BPGM activity in the resulting culture was measured. Comparing the data with those obtained in the culture of the cells transformed with pTRP-BPGM1, the activity level in the expression of BPGM in the former was about 5-fold the latter. As is obvious from the typical data in SAD-PAGE (in Fig. 2), the thick band was seen at the position of about 30,000 that corresponds to the molecular size of the E subunit of BPGM.

Fig. 2 shows the data as obtained by analyzing through SDS-PAGE the purification of the recombinant human BPGM from the cell extract of *E. coli* (pTRP-BPGM2), in which the lane 1 indicates the protein (20 μg) in the cell extract, the lane 2 indicates the protein (20 μg) in the heat-treated supernatant, the lane 3 indicates the protein (2 μg) in the eluate from the Blue-Sepharose column, the lane M indicates the IgG-binding molecule marker, and the arrow indicates the migration position as estimated from the molecular size (about 30,000) of the E subunit of BPGM.

Escherichia coli (pTRP-BPGM1) and Escherichia coli (pTRP-BPGM2) were deposited under No. FERM BP-5760 and No. FERM BP-5761, respectively, in The National Institute of Bioscience and Human-Technology, Agency of Industrial Science and Technology, Ministry of International Trade and Industry on November 28, 1996 under the Budapest Treaty.

(10) Purification of Recombinant Human BPGM:

The recombinant human BPGM was purified about 20-fold (see Table 1 below), according to the modification of the Calvin et al's method comprising heat treatment (at 55°C for 30 minutes) followed by affinity chromatography with Blue-Sepharose (pH 7.5). The yield was 35 %, and the relative activity was 12.3 U/mg. The amount produced was 1.0 g of the enzymatic protein per kg of wet cells. The purified BPGM showed a single band in SDS-PAGE at the position of a molecular size of about 30,000 (see Fig. 2).

Table 1 - Preparation of Recombinant Human BPGM

| Step, Fraction | Total Protein (mg) | Total Activity (KU) | Activity Obtained (%) | Relative Activity (U/mg) | Degree of Purification (times) |
|---|---|---|---|---|---|
| Extraction: Crude Extract (a) | 21820 | 5650 | (100) | 0.3 | (1.0) |
| Heat Treatment (55°C 30 min): Supernatant | 21600 | 2102 | 82 | 0.6 | 2.3 |
| Blue-Sepharose (pH 7.5): Eluate (b) | 132 | 1620 | 35 | 12.3 | 41 |

(a)   Obtained from 200 g of wet cells of recombinant E. coli.

(b) The elution was conducted with 10 mM Tris-HCl (pH 7.5) containing 1.0 M NaCl and 2 mM 2-phosphoglycerate.

EP 0 785 217 A1

(11) Preparation of Specific Polyclonal Antibody to Recombinant Human BPGM:

Using the recombinant human BPGM (25 mg), a specific rabbit polyclonal antibody was prepared.

(i) Preparation of IgG:

Using Protein A Sepharose, prepared was about 740 mg of IgG from 62 ml of rabbit antiserum.

(ii) Preparation of Specific IgG:

The IgG prepared above was put into a B-type PGAM-bound column, through which were obtained a non-adsorbed fraction and an adsorbed fraction. Pure preparations of human M-type PGAM, B-type PGAM and BPGM of 0.5 μg each were separately bound onto solid phases. These were individually reacted with each of these fractions (0.01 μg each) for EIA. The results are shown in Table 2 below. As in this, the non-adsorbed fraction had high affinity for PBGM but low affinity for M-type and B-type PGAM. Thus, this was a BPGM-specific IgG (hereinafter referred to as BPGM IgG). On the other hand, the adsorbed fraction was an IgG which reacted with all of BPGM and M-type and B-type PGAM (hereinafter referred to as MM/BB/BPGM IgG).

Thus, the addition of the IgG (370 mg) as prepared from rabbit anti-BPGM antiserum to the B-type PGAM-bound column (100 ml) gave 264 mg of BPGM IgG and 85 mg of MM/BB/BPGM IgG.

Table 2

| Reactivity in EIA of Specific IgG as Prepared in Recombinant Antigen-immobilized Column | | | |
|---|---|---|---|
| | $A_{492}$ | | |
| | BPGM-bound Plate | B-type PGAM-bound Plate | M-type PGAM-bound Plate |
| B-type PGAM-bound Column: | | | |
| Non-adsorbed Fraction | 0.520 | 0.004 | 0.000 |
| Adsorbed Fraction | 1.221 | 0.609 | 0.690 |

There are known some reports relating to rabbit anti-serum to human BPGM. However, there is no report relating to a specific antibody with no cross-reactivity with human PGAM. B-type PGAM having 53.8 % homology to BPGM in terms of the amino acid sequence exists in red blood cells and plasma. Therefore, in order to quantify BPGM through immunoassay, a specific antibody to human BPGM having no cross-reactivity with B-type PGAM must be prepared. According to the present invention, it has been demonstrated that a polyclonal IgG, which is specific to human BPGM and which does not react with human M-type and B-type PGAM, can be prepared from rabbit antiserum as immunized with an antigen of a purified, recombinat human PEGM, through two affinity chromatography processes.

(12) Principle of Quantitative Determination of Human BPGM by EIA:

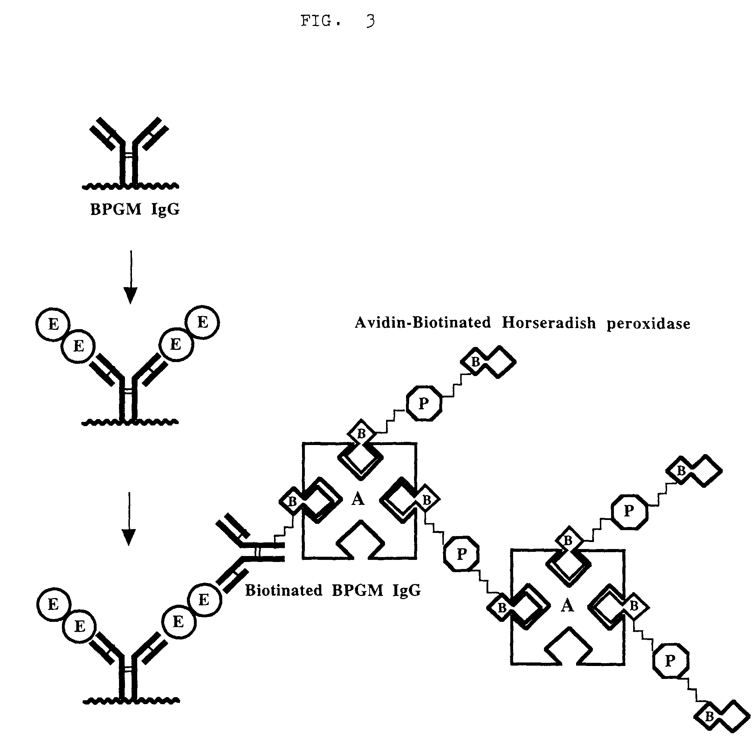

Referring to Fig. 3 showing the principle of ABC assay, BPGM in a sample was quantified. In Fig. 3, A means avidin, B means biotin, E means the subunit of BPGM, and P means horseradish peroxidase.

A solid-phase BPGM IgG was reacted with a BPGM sample, and then with a biotinated BPGM IgG. Next, this was reacted with avidin-biotinated horseradish peroxidase, and the amount of BPGM in the sample was obtained from the data of the reaction with the peroxidase.

(13) Preparation of Biotinated Antibody:

(i) BPGM IgG (1.65 mg/ml) was dialyzed against 100 mM $NaHCO_3$ buffer (pH 9.0) to prepare an antibody solution.
(ii) NHS-LC-Biotin II (produced by PIERCE Co.) was dissolved in dimethylformamide at a concentration of 30 mg/ml to prepare a biotinating reagent.
(iii) The biotinating reagent was added to the antibody solution in an amount of 10 μl per ml of the antibody solution, and well mixed.
(iv) These were reacted in ice for 2 hours.
(v) The reaction mixture was dialyzed against PBS buffer (8.77 g/liter NaCl, 1.56 g/liter $NaH_2PO_4 \cdot 2H_2O$, pH 7.2) to thereby remove the non-reacted biotinating reagent.

(14) Quantitative Determination of Human BPGM by EIA:

(i) BPGM IgG (10 μg/ml) as diluted with PBS buffer was put into a 96-well microtiter plate in an amount of 50 μl/well, and incubated overnight at 4°C.
(ii) The solution was removed from each well, which was then blocked with PBS buffer containing 0.1 % BSA (PBS-BSA), for 1 hour at room temperature.
(iii) This was washed with PBS buffer containing 0.1 % Tween 20 (PBS-Tween) for a total of 5 times.
(iv) A standard sample (a known amount of recombinant human BPGM) as diluted with PBS-BSA or a hemolyzed sample was added to this, in an amount of 50 μl/well, and incubated for 2 hours at room temperature.
(v) This was washed with PBS-Tween for a total of 5 times.
(vi) A Vectastain ABC Kit (produced by Vector Laboratories Co.) as diluted 150-fold with PBS-BSA was added to this in an amount of 50 μl/well, and incubated for 1 hour at room temperature.
(vii) This was washed with PBS-Tween for a total of 5 times.
(viii) One mg/ml o-phenylenediamine dihydrochloride (produced by Wako Pure Chemical Industries Co.), 1 μl/ml $H_2O_2$, and 0.1 M phosphoric acid buffer (pH 6.0) were added to this, in an amount of 100 μl/well each, and incubated for 3 minutes at room temperature.
(ix) 4 N HCl was added to this in an amount of 100 μl/well, by which the reaction was stopped.
(x) Using a plate reader, the absorbance at 492 nm (with side wavelength of 630 nm) was measured.

(15) Formation of Calibration Curve with Standard Sample:

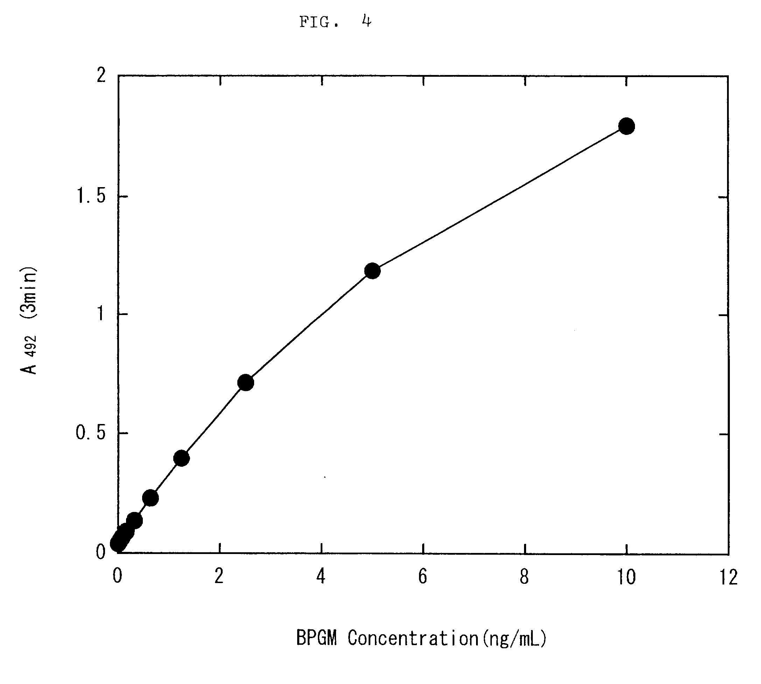

A series of dilutions of recombinant human BPGM with PBS-BSA were prepared, each having a BPGM concentration of 10, 5, 2.5, 1.25, 0.63, 0.32, 0.16, 0.08, 0.04 ng/ml. The BPGM content of each dilution was qualified according to EIA. The absorbance at 492 nm ($A_{492}$) was plotted relative to the varying recombinant BPGM concentrations to give the calibration curve as shown in Fig. 4.

(16) Cross-Reactivity with B-type PGAM:

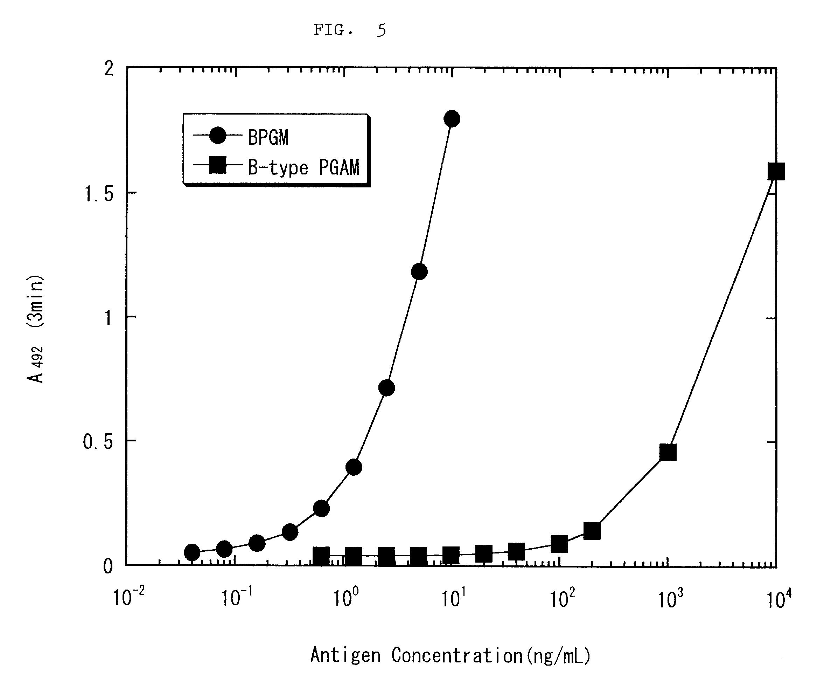

A series of dilutions of recombinant human B-type PGAM with PBS-BSA were prepared, each having a PGAM concentration of 10000, 1000, 200, 100, 10, 20, 10, 5, 2.5, 1.25, 0.63 ng/ml. Using these, the cross-reactivity with the B-type PGAM in the quantitative determination of BPGM by EIA was determined. The data are shown in Fig. 5. As in this, it was found that there is no cross-reaction with B-type PGAM within the range where PBGM is quantified (from 0 to 10 ng/ml).

(17) Preparation of Hemolyzed Sample:

(i) 0.5 ml of 3.8 % sodium citrate was added to 4.5 ml of a whole blood sample, gently stirred, and then centrifuged (2,500 rpm, 5 minutes).
(ii) 3.0 ml of 0.9 % NaCl was added to the thus-separated red blood cells, gently stirred, and then centrifuged (2,500 rpm, 5 minutes).
(iii) The resulting supernatant was removed. Then, the process of (ii) was repeated three times.
(iv) 5 ml of pure water and 1.25 ml of toluene were added to the thus-separated red blood cells, fully mixed in a mixture, and then centrifuged (2,500 rpm, 20 minutes).
(v) The hemolyzed sample layer was collected from the interlayer.

(18) Quantitative Determination of Human BPGM in Hemolyzed Sample:

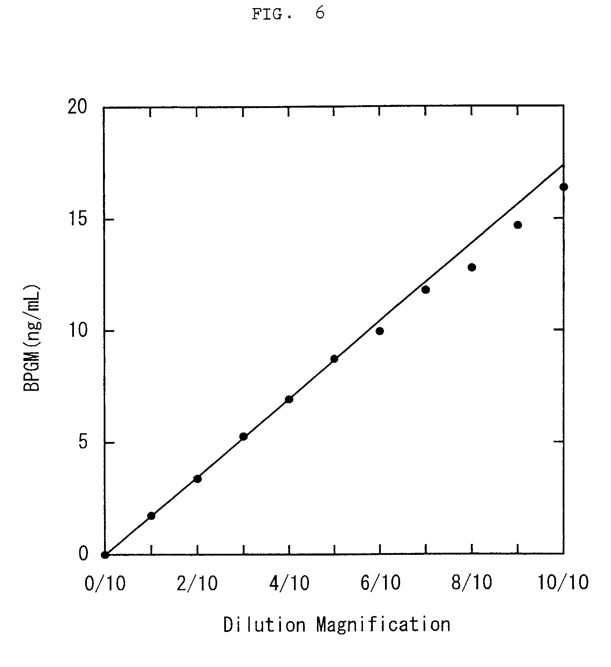

The hemolyzed sample prepared from healthy human blood was diluted 2000-fold with PBS-BSA. Based on this, 10/10 sample, a series of dilutions with PBS-BSA were prepared, 9/10, 8/10, 7/10, . . . 1/10. The BPGM content of each dilution was quantified according to EIA. For each dilution, the BPGM concentration as obtained from the calibration curve was plotted to give the graph of Fig. 6. As in this, the linearity in dilution up to 7/10 samples was obtained. This demonstrates that the BPGM content of such a hemolyzed sample can also be determined like that in the standard sample.

(19) Test for Addition and Recovery of Recombinant Human BPGM:

(i) Three samples were prepared in the manner mentioned below, of which the BPGM content was measured by EIA.

(A) Hemolyzed Sample + Recombinant Human BPGM:

13.9 µl of recombinant human BPGM (216 µg/ml) was added to 86.1 µl of the hemolyzed sample, which was diluted 10000-fold with PBS-BSA.

(B) Recombinant Human BPGM:

13.9 µl of recombinant human BPGM (216 µg/ml) was added to 86.1 µl of PBS-BSA, which was diluted 10000-fold with PBS-BSA.

(C) Hemolyzed Sample:

The hemolyzed sample was diluted 10000-fold with PBS-BSA.

(ii) From the calibration curve, the BPGM content of each of these (A), (B), (C) was obtained, and the percentage of recovery of BPGM was calculated according to the following equation.

Percentage of Recovery (%)

$$= \{[(A) - (C) \times 0.861]/B\} \times 100$$

As a result, (A) = 5.99 ng/ml; (B) = 2.52 ng/ml; (C) = 3.29 ng/ml; and the percentage of recovery was 125 %.

(20) Measurement of Influence of Interference Substances:

Reagents

Interference Check A (International Reagents Co.)

Bilirubin F (Free Bilirubin), Bilirubin C (Conjugated Bilirubin), Hemoglobin and Formazin

Interference Check RF (International Reagents Co.)

Rheumatoid Factor

Procedure

Test samples prepared as under-mentioned were diluted 10,000-fold with PBS-BSA and subjected to measurement of BPGM according to EIA.

A. Preparation of Test Samples to which Bilirubin F, Bilirubin C, Hemoglobin or Formazin was added:

1). Sample ② in Table 3 (Preparation of BPGM Sample) was thawed at room temperature to give a hemolysis-treated sample (abbreviated as a hemolyzed sample hereinafter). The hemolyzed sample was added to tubes in an amount of 180 µl/tube each. A solution of an interference substance having a concentration 10 times the maximum test concentration thereof was added to said 180 µl of the hemolyzed sample in an amount of 20 µl to give Solution (a), that is, Hemolyzed sample + Interference sample. Separately, PBS-BSA was added to said 180 µl of the hemolyzed sample in an amount of 20 µl to give Solution (b), that is, Hemolyzed sample + PBS-BSA.

2). The thus-obtained Solution (a) and Solution (b) were mixted with each other in the ratios as shown in Table 4 to give a series of dilutions of each interference substance.

B. Preparation of Test Samples to which Rheumatoid Factor was added:

A solution of Rheumatoid factor in the hemolyzed sample, which solution has a maximum test concentration of Rheumatoid factor, was prepared and mixed with the hemolyzed sample in the ratios as shown in Table 4 to give a series of dilutions of Rheumatoid factor.

Results

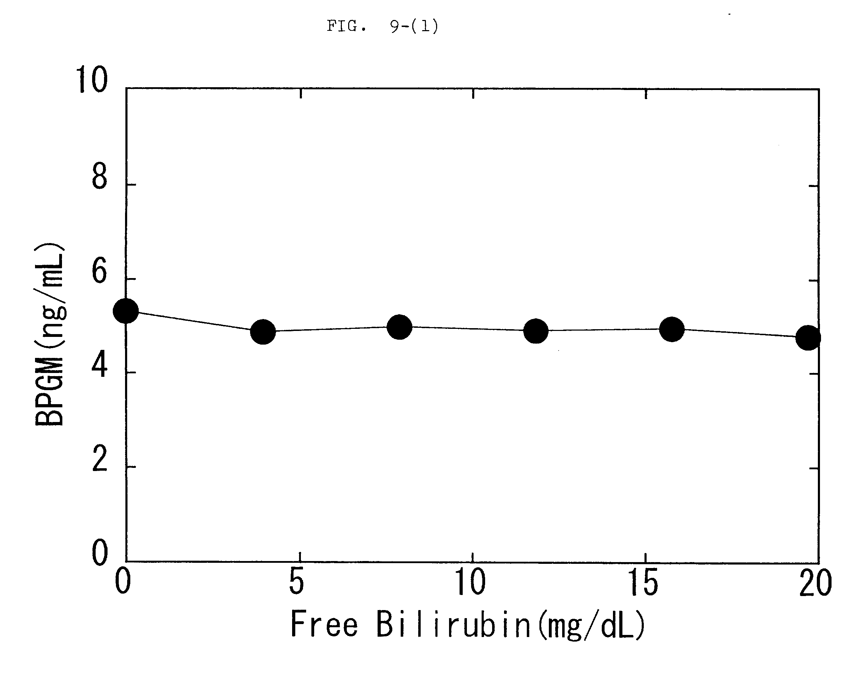

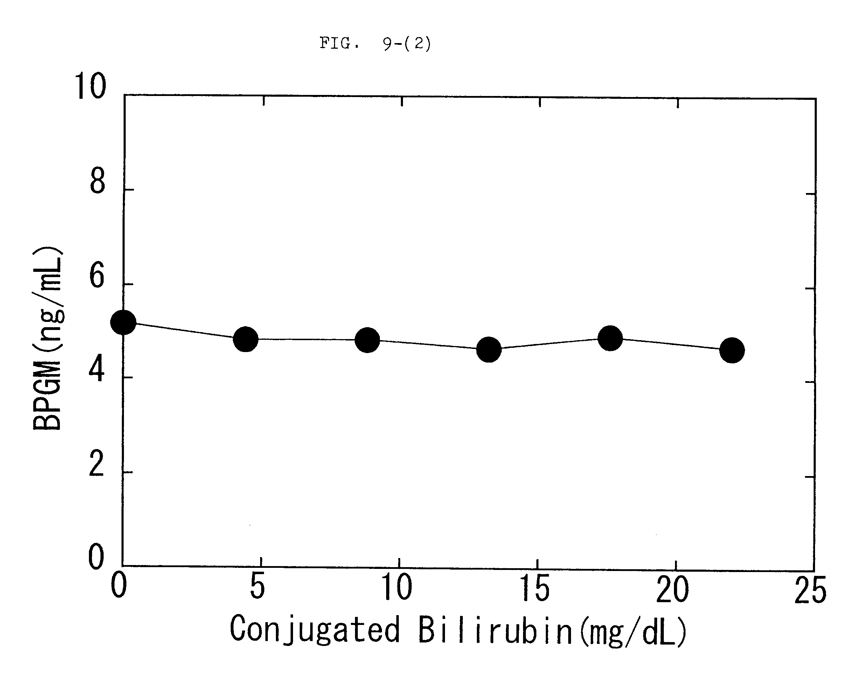

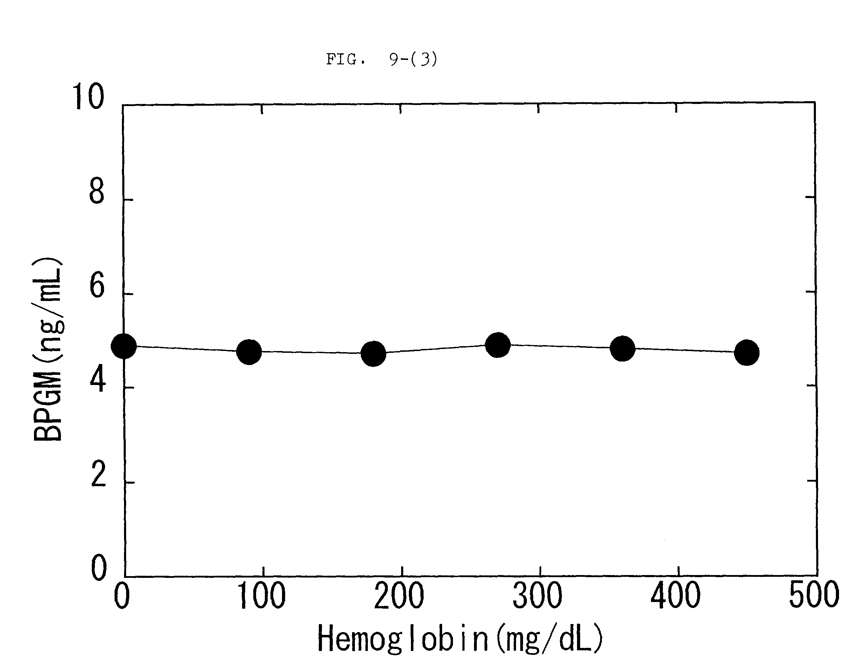

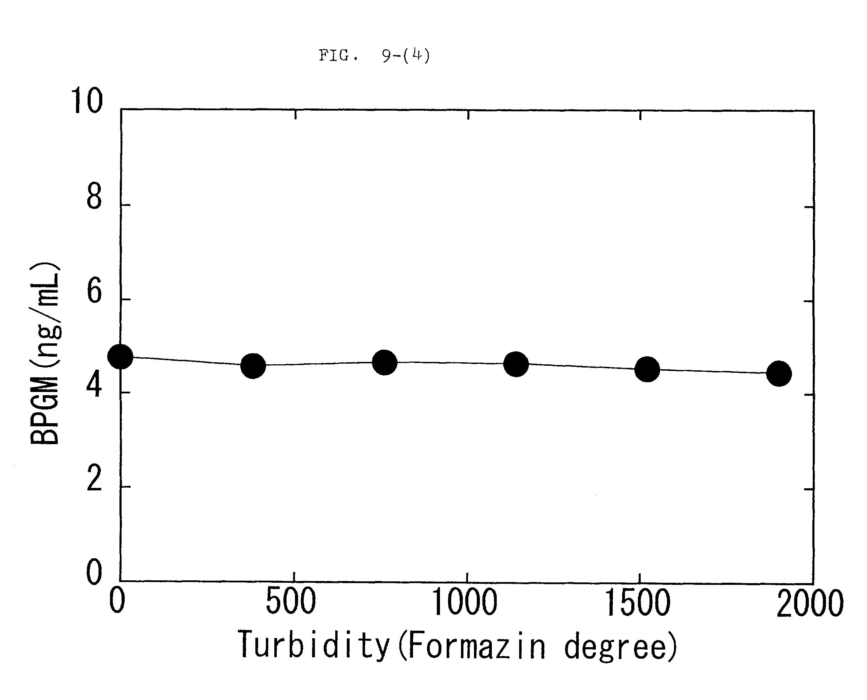

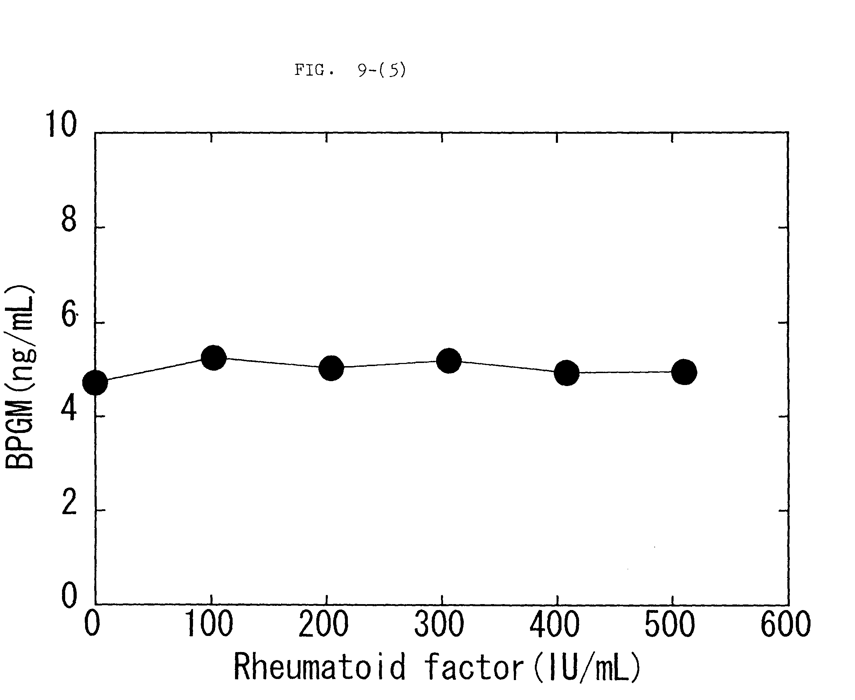

Measurement results are shown in Fig. 9-(1) to Fig. 9-(5).

Table 3  Preparation of BPGM Sample

EDTA-2K Blood (collected using VENOJECT Blood
              Collecting System by Terumo Co., Ltd.)
      |
   Cooling
   with ice

200 µL          Counting number of erythrocytes and
                measuring hematocrit

Adding 1.0 mL of
0.6 N perchloric   2.8 mL
acid cooled with
ice

Stirring
vigorously      Centrifuging (2,500 rpm, 5 min)

Freezing (-80°C)

Sample ①    Plasma          Haemocytes

            Freezing        Adding 2.0 mL of 0.9%
            (-80°C)         NaCl cooled with ice

                            Mixing gently

                            Centrifuging                Washing 3 times
                            (2,500 rpm, 5 min)

                            Removing the
                            supernatant

                            Adding 1.5 mL of 0.9% NaCl
                            cooled with ice

                            Stirring gently and
                            counting number of erythrocytes

                            Freezing (-80°C)

                            Sample ②

<u>Note</u>

Sample ① : Measurement of 2,3-BPG

Sample ② : Measurement of BPGM amount and BPGM activity

EDTA-2K Blood: Whole blood collected by using EDTA-2K
               as anticoagulant
               EDTA-2K is ethylenediaminetetraacetic acid
               dipotassium salt.

Table 4

| Bilirubin F | 0mg/dL | 3.9mg/dL | 7.9mg/dL | 11.8mg/dL | 15.8mg/dL | 19.7mg/dL |
|---|---|---|---|---|---|---|
| Hemolyzed sample + Interference sample | 0 μL | 10 μL | 20 μL | 30 μL | 40 μL | 50 μL |
| Hemolyzed sample + PBS-BSA | 50 μL | 40 μL | 30 μL | 20 μL | 10 μL | 0 μL |

| Bilirubin C | 0mg/dL | 4.4mg/dL | 8.8mg/dL | 13.2mg/dL | 17.6mg/dL | 22.0mg/dL |
|---|---|---|---|---|---|---|
| Hemolyzed sample + Interference sample | 0 μL | 10 μL | 20 μL | 30 μL | 40 μL | 50 μL |
| Hemolyzed sample + PBS-BSA | 50 μL | 40 μL | 30 μL | 20 μL | 10 μL | 0 μL |

| Hemoglobin | 0mg/dL | 90mg/dL | 180mg/dL | 270mg/dL | 360mg/dL | 450mg/dL |
|---|---|---|---|---|---|---|
| Hemolyzed sample + Interference sample | 0 μL | 10 μL | 20 μL | 30 μL | 40 μL | 50 μL |
| Hemolyzed sample + PBS-BSA | 50 μL | 40 μL | 30 μL | 20 μL | 10 μL | 0 μL |

| Formazin | 0 D.* | 380 D.* | 760 D.* | 1140 D.* | 1520 D.* | 1900 D.* |
|---|---|---|---|---|---|---|
| Hemolyzed sample + Interference sample | 0 μL | 10 μL | 20 μL | 30 μL | 40 μL | 50 μL |
| Hemolyzed sample + PBS-BSA | 50 μL | 40 μL | 30 μL | 20 μL | 10 μL | 0 μL |

* D. means Degree.

| Rheumatoid factor | 0 IU/mL | 102 IU/mL | 204 IU/mL | 306 IU/mL | 408 IU/mL | 510 IU/mL |
|---|---|---|---|---|---|---|
| Hemolyzed sample + Interference sample | 0 μL | 10 μL | 20 μL | 30 μL | 40 μL | 50 μL |
| Hemolyzed sample | 50 μL | 40 μL | 30 μL | 20 μL | 10 μL | 0 μL |

EP 0 785 217 A1

(21) Measurement of Hemolyzed Sample of Healthy Human:

A. Quantitative Determination of BPGM:

Procedure

Sample ② in Table 3 was thawed at room temperature to give a hemolyzed sample. This sample was diluted 10,000-fold with PBS-BSA and subjected to quantitative determination of BPGM according to EIA.

Processing of Measurements obtained

The amount of BPGM (μg/ml whole blood) was calculated according to the following formula:

$$\text{BPGM (μg/ml whole blood)} =$$

$$\text{BPGM (μg/ml hemolyzed sample)} \times \frac{\text{Number of erythrocytes in the whole blood ( x } 10^4/\mu l)}{\text{Number of erythrocytes in the hemolyzed sample ( x } 10^4/\mu l)}$$

B. Measurement of BPGM Activity:

Conditions of Measurement

Temperature: 37°C
Wave length: 340 nm

Procedure

1). Sample ② in Table 3 was thawed at room temperature to give a hemolyzed sample. This sample was diluted 10-fold with 50 mM Tris-HCl buffer (pH 8.0). The thus-obtained dilution was used as a sample for measurement of BPGM activity.
2). The subsequent procedure was carried out according to Calvin et al's method.

Processing of Measurements obtained

BPGM activity (U/ml whole blood) was calculated according to the following formula:

$$\text{BPGM Activity (U/ml whole blood)} =$$

$$\text{BPGM Activity (U/ml hemolyzed sample)} \times \frac{\text{Number of erythrocytes in the whole blood ( x } 10^4/\mu l)}{\text{Number of erythrocytes in the hemolyzed sample ( x } 10^4/\mu l)}$$

C. Calculation of Specific Activity:

Specific Activity was calculated according to the following formula:

$$\text{Specific Activity (U/mg protein)} = \frac{\text{BPGM Activity (U/ml whole blood)}}{\text{BPGM (μg/ml whole blood)} \times \dfrac{1}{1,000}}$$

D. Quantitative Determination of 2,3-BPG:

Reagent

Kit: 2,3-DPG Test "BMY" (Boehringer)

Conditions of Measurement

Temperature: 25°C
Wave length: 340 nm

Procedure

1). Sample ① in Table 3 was thawed to give a test sample for measurement of 2,3-BPG. This test sample was added to 2 ml-tubes in an amount of 0.6 ml/tube each and centrifuged (2,500 rpm, 5 min).

2). 0.4 ml of the thus-obtained supernatant was added to a separate 2 ml-tube, and mixed with 50 μl of 2.5 M $K_2CO_3$ added thereto for neutralization.

3). The resultant mixture was cooled with ice for 30 minutes.

4). The precipitated potassium perchlorate was removed by centrifugation (2,500 rpm, 5 min). 0.1 ml of the thus-obtained supernatant was used as a test sample for measurement of 2,3-BPG. 0.1 ml of pure water was used as control.

5). The subsequent steps were carried out according to the procedure for the kit.

Processing of measurements obtained

The amount of absorbance change of the test sample ($\Delta E$) and that of the control ($\Delta Eb$) were measured, and the amount of 2,3-BPG ($\mu$ mole/ml whole blood) was calculated according to the following formula:

$$11.70 \times (\Delta E - \Delta Eb)$$

E. Results:

The data obtained are shown in Table 5.

Table 5

| Sample | BPGM $\mu$ g/mL whole blood* | BPGM Activity U/mL whole blood* | Specific Activity U/mg protein | 2,3-BPG $\mu$ mol/mL whole blood* |
|---|---|---|---|---|
| 1 | 66.0 | 1.29 | 19.6 | 2.32 |
| 2 | 46.7 | 1.04 | 22.2 | 1.94 |
| 3 | 62.6 | 1.34 | 21.5 | 2.45 |
| 4 | 69.4 | 1.47 | 21.2 | 2.28 |
| 5 | 53.4 | 1.02 | 19.1 | 1.90 |
| 6 | 48.6 | 1.11 | 22.8 | 2.20 |
| 7 | 52.3 | 1.09 | 20.8 | 1.78 |
| Mean | 57.0 | 1.20 | 21.0 | 2.12 |

\* The whole blood contains EDTA-2K.

Advantages of the Invention:

According to the present invention, it has become possible to efficiently produce high-purity, human recombinant BPGM through genetic engineering operation. As a result of this, it has become possible, for the first time, to prepare a specific antibody to the human recombinant BPGM.

Since the novel antibody of the present invention has extremely high specificity to BPGM, it can be used in specific, high-sensitive detection and quantitative determination of BPGM in red blood cells, plasma and other samples according to immunoassay such as EIA.

Accordingly, constructing and using a kit comprising the novel antibody and other reagents necessary for immunoassay, it is possible to measure the BPGM content of various samples and to diagnose various diseases associated with 2,3-BPG.

Sequences

In the SEQUENCE LISTING which follows,

- SEQ ID N° : 1 and 2 relate to the nucleotide and amino acid sequences of primer P1,
- SEQ ID N° : 3 and 4 relate to the nucleotide and amino acid sequences of primer P2,
- SEQ ID N° : 5 relates to the nucleotide sequence of primer P3,
- SEQ ID N° : 6 and 7 relate to the nucleotide and amino acid sequences of primer P4,
- SEQ ID N° : 8 and 9 relate to the nucleotide and amino acid sequences of primer P5, and
- SEQ ID N° : 10 and 11 relate to the nucleotide sequences of the Figure 8 gene cassette materials for tryptophan promoter.

## Claims

1. An antibody to bisphosphoglycerate mutase (BPGM).

2. The antibody as claimed in claim 1, which is separated and purified from an animal anti serum as immunized with an antigen of a human recombinant BPGM.

3. The antibody as claimed in claim 2, wherein the human recombinant BPGM is obtained by inserting a human BPGM gene into an expression vector having a tryptophan promoter, then transforming microorganisms with the resulting expression plasmid, and expressing the intended, recombinant human BPGM in the resulting transformants.

4. The antibody as claimed in claim 3, wherein the human BPGM gene is prepared through mPCR using a human placenta-derived chromosomal DNA as the template.

5. The antibody as claimed in claim 3 or 4, wherein the BPGM expressing plasmid is any of BPGM expressing plasmids pTRP-BPGM1 and/or pTRP-BPGM2 as obtained by inserting a human BPGM gene into an expression vector of pTRP.

6. A method of immunoassay for BPGM, in which is used the antibody as claimed in any one of claims 1 to 5.

7. The method as claimed in claim 6, which the immunoassay is one member selected from the group consisting of immunostaining, radioimmunoassay, enzyme immunoassay and fluorescent immunoassay.

8.	A kit for measuring BPGM, which comprises the antibody as claimed in any one of claims 1 to 5.

FIG. 1

## Nucleotide Sequences and Amino Acid Sequences of Primers

```
                          MetSerLysTyrLysLeuIle
P1 (29 mer)        5' CCGGAATTC ATGTCCAAGTACAAACTTAT- 3'
                      EcoRI


                   AlaLeuLeuLysHisLeuGlu    GlyIleSerAspGluAsp
P2 (35 mer)        3' --TGAGGATTTTGTGGACCTTC ↓ CATAGAGTCTACTTC--  5'


                                           *************** Ile
P3 (20 mer)                          5' GTATCTCAGATGAAGACATC  3'


                   ValLysGlnAlaLysLysTer
P4 (29 mer)        3' -ACTTTGTTCGATTTTTTATC CCTAGGCCC-  5'
                                          BamHI
```

```
                          MetSerLysTyrLysLeuIleMetLeuArgHisGly
P5 (45 mer)        5' CCGGAATTC ATGTCAAAATACAAACTGATCATGTTAAGACATGGA 3'
                      EcoRI         #  #        #  #
```

FIG. 2

FIG. 3

BPGM IgG

Avidin-Biotinated Horseradish peroxidase

Biotinated BPGM IgG

FIG. 4

EP 0 785 217 A1

FIG. 5

EP 0 785 217 A1

FIG. 6

FIG. 7

FIG. 8

## Gene Cassette for trp Promoter

*Hind*III                                                         *Pst*I

5' AGCTTCCCTGTTGACAATTAATCATCGAACTAGTTAACTAGTACGCAATGGCACGTAAAAAGGGTAGAATTCCTGCA 3'

    AGGGACAACTGTTAATTAGTAGCTTGATCAATTGATCATGCGTTCAAGTGACTTTTTCCCATCTTAAGG

*Eco*RI

EP 0 785 217 A1

FIG. 9-(1)

EP 0 785 217 A1

FIG. 9-(2)

FIG. 9-(3)

FIG. 9-(4)

EP 0 785 217 A1

FIG. 9-(5)

**European Patent Office**

## EUROPEAN SEARCH REPORT

Application Number

EP 97 40 0098

### DOCUMENTS CONSIDERED TO BE RELEVANT

| Category | Citation of document with indication, where appropriate, of relevant passages | Relevant to claim | CLASSIFICATION OF THE APPLICATION (Int.Cl.6) |
|---|---|---|---|
| X | THE EMBO JOURNAL, vol. 5, no. 9, September 1986, OXFORD, GB, pages 2275-2283, XP000671743 V. JOULIN ET AL.: "Molecular cloning and sequencing of the human erythrocyte 2,3-biphosphoglycerate mutase cDNA: revised amino acid sequence." * page 2275, right-hand column, line 39 - line 51 * * page 2281, right-hand column, line 40 - line 46 * * figures 2,3 * | 1-8 | C07K16/40 G01N33/577 G01N33/573 //C12N9/90 |
| X | BIOCHIMICA ET BIOPHYSICA ACTA, vol. 742, no. 1, 12 January 1983, AMSTERDAM, NL, pages 243-249, XP000673320 R. ROSA ET AL.: "Partial characterization of the inactive mutant form of human red cell biphosphoglyceromutase and comparison with an alkylated form." * page 245, right-hand column, line 5 - line 42 * | 1-8 | |
| A | BIOMEDICA ET BIOCHIMICA ACTA, vol. 46, no. 2-3, 1987, GERMANY, pages S126-S130, XP000671745 M. COHEN-SOLAL ET AL.: "Molecular cloning of the human 2,3-biphosphoglycerate mutase cDNA and revised amino acid sequence." * summary * * figures * | 1-8 | |
| A | EP 0 365 894 A (FARMITALIA CARLO ERBA S.R.L.) 2 May 1990 * claim 1 * | 3,5 | |

**TECHNICAL FIELDS SEARCHED (Int.Cl.6)**

C12N
C07K

-/--

The present search report has been drawn up for all claims

| Place of search | Date of completion of the search | Examiner |
|---|---|---|
| THE HAGUE | 12 May 1997 | Nooij, F |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EP 0 785 217 A1

European Patent
Office

**EUROPEAN SEARCH REPORT**

Application Number

EP 97 40 0098

| | DOCUMENTS CONSIDERED TO BE RELEVANT | | |
|---|---|---|---|
| Category | Citation of document with indication, where appropriate, of relevant passages | Relevant to claim | CLASSIFICATION OF THE APPLICATION (Int.Cl.6) |
| A | PROCEEDINGS OF THE NATIONAL ACADEMY OF SCIENCES OF THE USA, vol. 91, no. 9, 26 April 1994, WASHINGTON, DC, USA, pages 3593-3597, XP002030756 M. GAREL ET AL.: "A recombinant biphosphoglycerate mutase variant with acid phosphatase homology degrades 2,3-diphosphoglycerate." * abstract * ----- | 1-8 | |
| | | | TECHNICAL FIELDS SEARCHED (Int.Cl.6) |

The present search report has been drawn up for all claims

| Place of search | Date of completion of the search | Examiner |
|---|---|---|
| THE HAGUE | 12 May 1997 | Nooij, F |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

............................................................................

& : member of the same patent family, corresponding document

SEQUENCE LISTING


(1) GENERAL INFORMATION:

    (i) APPLICANT:
        (A) NAME: Oriental Yeast Co., Ltd.
        (B) STREET: 6-10, Azusawa 3-chome, Itabashi-ku,
        (C) CITY: TOKYO
        (E) COUNTRY: Japan
        (F) POSTAL CODE (ZIP): none

    (ii) TITLE OF INVENTION: METHOD OF IMMUNOASSAY FOR HUMAN BPGM

    (iii) NUMBER OF SEQUENCES: 11

    (iv) COMPUTER READABLE FORM:
        (A) MEDIUM TYPE: Floppy disk
        (B) COMPUTER: IBM PC compatible
        (C) OPERATING SYSTEM: PC-DOS/MS-DOS
        (D) SOFTWARE: PatentIn Release #1.0, Version #1.30 (EPO)

    (vi) PRIOR APPLICATION DATA:
        (A) APPLICATION NUMBER: JP 8-24897
        (B) FILING DATE: 19-JAN-1996

    (vi) PRIOR APPLICATION DATA:
        (A) APPLICATION NUMBER: JP unknown
        (B) FILING DATE: 04-DEC-1996


(2) INFORMATION FOR SEQ ID NO:1:

    (i) SEQUENCE CHARACTERISTICS:
        (A) LENGTH: 29 base pairs
        (B) TYPE: nucleic acid
        (C) STRANDEDNESS: single
        (D) TOPOLOGY: linear

    (ii) MOLECULE TYPE: DNA (genomic)




    (xi) SEQUENCE DESCRIPTION: SEQ ID NO:1:

CCGGAATTCA TGTCCAAGTA CAAACTTAT                29

(2) INFORMATION FOR SEQ ID NO:2:

    (i) SEQUENCE CHARACTERISTICS:
        (A) LENGTH: 7 amino acids
        (B) TYPE: amino acid
        (C) STRANDEDNESS: single
        (D) TOPOLOGY: linear

    (ii) MOLECULE TYPE: peptide




    (xi) SEQUENCE DESCRIPTION: SEQ ID NO:2:

    Met Ser Lys Tyr Lys Leu Ile

(2) INFORMATION FOR SEQ ID NO:3:

    (i) SEQUENCE CHARACTERISTICS:
        (A) LENGTH: 35 base pairs
        (B) TYPE: nucleic acid
        (C) STRANDEDNESS: single
        (D) TOPOLOGY: linear

    (ii) MOLECULE TYPE: DNA (genomic)

    (xi) SEQUENCE DESCRIPTION: SEQ ID NO:3:

CTTCATCTGA GATACCTTCC AGGTGTTTTA GGAGT        35

(2) INFORMATION FOR SEQ ID NO:4:

    (i) SEQUENCE CHARACTERISTICS:
        (A) LENGTH: 13 amino acids
        (B) TYPE: amino acid
        (C) STRANDEDNESS: single
        (D) TOPOLOGY: linear

    (ii) MOLECULE TYPE: peptide

    (xi) SEQUENCE DESCRIPTION: SEQ ID NO:4:

    Ala Leu Leu Lys His Leu Glu Gly Ile Ser Asp Glu Asp
    1             5                    10

(2) INFORMATION FOR SEQ ID NO:5:

    (i) SEQUENCE CHARACTERISTICS:
        (A) LENGTH: 20 base pairs
        (B) TYPE: nucleic acid
        (C) STRANDEDNESS: single
        (D) TOPOLOGY: linear

    (ii) MOLECULE TYPE: DNA (genomic)

    (xi) SEQUENCE DESCRIPTION: SEQ ID NO:5:

GTATCTCAGA TGAAGACATC        20

(2) INFORMATION FOR SEQ ID NO:6:

    (i) SEQUENCE CHARACTERISTICS:
        (A) LENGTH: 29 base pairs
        (B) TYPE: nucleic acid
        (C) STRANDEDNESS: single
        (D) TOPOLOGY: linear

    (ii) MOLECULE TYPE: DNA (genomic)

(xi) SEQUENCE DESCRIPTION: SEQ ID NO:6:

CCCGGATCCC TATTTTTTAG CTTGTTTCA                                    29

(2) INFORMATION FOR SEQ ID NO:7:

    (i) SEQUENCE CHARACTERISTICS:
        (A) LENGTH: 6 amino acids
        (B) TYPE: amino acid
        (C) STRANDEDNESS: single
        (D) TOPOLOGY: linear

    (ii) MOLECULE TYPE: peptide

    (xi) SEQUENCE DESCRIPTION: SEQ ID NO:7:

    Val Lys Gln Ala Lys Lys
    1               5

(2) INFORMATION FOR SEQ ID NO:8:

    (i) SEQUENCE CHARACTERISTICS:
        (A) LENGTH: 45 base pairs
        (B) TYPE: nucleic acid
        (C) STRANDEDNESS: single
        (D) TOPOLOGY: linear

    (ii) MOLECULE TYPE: DNA (genomic)

    (xi) SEQUENCE DESCRIPTION: SEQ ID NO:8:

CCGGAATTCA TGTCAAAATA CAAACTGATC ATCTTAAGAC ATGGA        45

(2) INFORMATION FOR SEQ ID NO:9:

    (i) SEQUENCE CHARACTERISTICS:
        (A) LENGTH: 12 amino acids
        (B) TYPE: amino acid
        (C) STRANDEDNESS: single
        (D) TOPOLOGY: linear

    (ii) MOLECULE TYPE: peptide

    (xi) SEQUENCE DESCRIPTION: SEQ ID NO:9:

    Met Ser Lys Tyr Lys Leu Ile Met Leu Arg His Gly
    1               5                   10

(2) INFORMATION FOR SEQ ID NO:10:

    (i) SEQUENCE CHARACTERISTICS:
        (A) LENGTH: 77 base pairs

```
(B)  TYPE: nucleic acid
(C)  STRANDEDNESS: single
(D)  TOPOLOGY: linear

   (ii)  MOLECULE TYPE: DNA (genomic)



      (xi)  SEQUENCE DESCRIPTION: SEQ ID NO:10:

   AGCTTCCCTG TTGACAATTA ATCATCGAAC TAGTTAACTA GTACGCAATG GCACGTAAAA 60

   AGGGTAGAAT TCCTGCA                                                 77

   (2)  INFORMATION FOR SEQ ID NO:11:

         (i)  SEQUENCE CHARACTERISTICS:
               (A)  LENGTH: 69 base pairs
               (B)  TYPE: nucleic acid
               (C)  STRANDEDNESS: single
               (D)  TOPOLOGY: linear

         (ii)  MOLECULE TYPE: DNA (genomic)



      (xi)  SEQUENCE DESCRIPTION: SEQ ID NO:11:

   GGAATTCTAC CCTTTTTCAG TGAACTTGCG TACTAGTTAA CTAGTTCGAT GATTAATTGT 60

   CAACAGGGA                                                          69
```